# EUROPEAN PATENT APPLICATION

(11) **EP 4 201 418 A1**
(43) Date of publication of application: **28.06.2023**
(21) Application number: 21217666.3
(22) Date of filing: 23.12.2021
(51) Int. Cl.: A61K 36/48, A61K 36/185, A61K 36/81, A61K 36/899, A61P 17/04, A23L 33/105, A61K 31/375, A61K 33/30, A61Q 7/00

(54) **HAIR CARE NUTRITIONAL SUPPLEMENT COMPOSITION**

(71) Applicant: ASC Regenity Ltd., London W1C 2PE (GB)
(72) Inventor: GIRI, Shibashish, 04109 LEIPZIG (DE)
(74) Representative: Touroude & Associates

(57) **Abstract**

The invention relates to a hair care oral nutritional supplement composition comprising at least fenugreek seed extract, ashwagandha root extract, bambusa bamboo extract and moringa seed extract, for reviving, stimulating and enhancing hair care safely, efficiently and naturally, preferably for prevention and reduction of hair loss, balding disorders, thinning problems. The invention relates to a bio-optimized combined approaches of botanical extracts with optimum dose of nature amino acid composition, required in hair bulb for biological hair care, to deeply detoxify, clean the root of hair follicle while delivering all formulated ingredients to the root of hair.

## Description

### Field of the Invention

The invention relates to a hair care oral nutritional supplement composition comprising at least fenugreek seed extract, ashwagandha root extract, bambusa bamboo extract and moringa seed extract, for reviving, stimulating and enhancing hair care safely, efficiently and naturally, preferably for prevention and reduction of hair loss, balding disorders, thinning problems. The invention relates to a bio-optimized combined approaches of botanical extracts with optimum dose of nature amino acid composition, required in hair bulb for biological hair care, to deeply detoxify, clean the root of hair follicle while delivering all formulated ingredients to the root of hair.

### Background of the Invention

Baldness is a societal problem for most. It often comes for the patient with mental disorders coming with hard feelings as lack of attractiveness, sexual potency and aging. This syndrome is complex, dependent on many factors and further, the psychological complications are huge.

Hair loss is a common problem in both males and females regardless of their age. There are several reasons for hair loss, whether genetic disposition, advancing age, imbalance of hormones and other physical body parameters, adverse drug effect, chemotherapy, effect of certain medical conditions, nutritional deficiency, environmental condition such as water pollution, air pollution, improper blood purification, excessive calcium deposition, scalp or hair trauma, microbial infections and any such causes.

Hair loss is a natural process but every hair follicle of hair should be recycled to produce new hair. Unnatural hair loss is mainly due to hormonal Dihydrotestosterone (DHT) imbalance, inadequate blood flow going to the head or to the root of the hair, lower oxygen and nutrients to scalp of the head.

For example, male hormone dihydrotestosterone (herein after referred as DHT) causes hair follicles to degrade and further shrink in size, resulting in weak hairs. DHT production in female is 15 times less compare with male. DHT is an androgenic hormone which is a sex steroid produced from testosterone of the gonads. DHT also shortens the anagen phase of the hair follicle growing cycle and enhances the telogen phase of hair falls out, thus over time, more hairs fall and hairs become thinner. Excess secretion of DHT and its accumulation around hair follicle of hair causes hair loss. Moreover, Excess accumulation of DHT around hair follicle stem cell pool prevents the oxygen delivery, nutrients supply, activation of hair follicle stem cells. The most common pharmacological management of this problem is topical Minoxidil and Finasteride taken orally. Minoxidil and Finasteride are FDA approved drugs which can block the formation of DHT, but which are also associated with several health risks. Minoxidil increases the blood flow and vascularity in the scalp when topically applied and Finasteride inhibits the enzyme 5-alpha-reductase preventing the conversion of testosterone to DHT when given orally. However, Minoxidil provides side effects such as scalp dryness, skin irritation, rashes, burning, redness, erythema, dryness, allergic contact dermatitis. Finasteride provides side effects such as impotence, decreased libido, erectile dysfunction and testicular pain, ejaculation disorders, breast enlargement or tenderness, headache, menstrual irregularity, dizziness, increased body hair growth, hypersensitivity reactions. Thus, it is time to provide new solutions to facilitate the life of the patients, and not obligate them to live with the above maj or side effects resulting from the use of these two FDA approved drugs.

In addition, a report by Goldman et al. (1996) evaluated whether male pattern baldness is associated with a deficiency in oxygen supply to body tissue. The results indicate that penetration of oxygen was lower in the bald frontal scalp than in hair-bearing temporal scalp area. As such, good blood supply to the scalp is essential to maintain normal cycle of hair growth.

A proper nutritional supplement can be a contributing factor in maintaining healthy hair. There are a variety of known dietary supplements which can affect human hair growth. Oral administration of food supplements products can be beneficial for maintaining healthy hair and for reviving, stimulating and enhancing hair growth.

Numerous attempts have been made in several prior art. Even though these innovations may be suitable for the specific purposes to which they address, however, they would not be as suitable for the purposes of the present invention.

For example, PCT patent application No. WO2007034323A1 discloses a composition to treat hair loss, balding, and thinning disorders or problems including fenugreek seed extract, flax lignans, saw palmetto berry extract, biotin; calcium d-pantothenic acid, nicotinamide, pyridoxine HCL, riboflavin, and folic acid.

Chinese patent application No. CN101085215A discloses a vein-relaxing and hair-tonic capsule comprising: fleece-flower root, mulberry seed, doddor seed, psoralea fruit, Chinese angelica root, walnut pulp, Siberian solomonseal rhizome, buthus martensi kirsch, black sesame seeds, licorice root and centipede.

Great Britain patent No. GB2484812A discloses an oral preparation for maintaining hair growth, strength and condition and reducing hair loss, comprising: pine bark extract, caffeine, curcumin, resveratrol horsetail extract L-cysteine, methylsulphonylmethane, biotin, vitamin B6, P5P, Zinc, Copper, and Magnesium. The composition may further comprise saw palmetto berries extract, beta sitosterol, green tea and taurine or iron, vitamin B12, marine cartilage and pantothenic acid.

U. S. Patent No. US10709659B1 discloses a composition and a method for promoting hair growth and reducing hair loss using bioactive extracts of curcumin, Withania somnifera, and saw palmetto. Tocotrienols, tocopherols, piperine extract, low molecular weight collagen, and hyaluronic acid are also optionally included in the composition.

U. S. Published Patent Application No. discloses a pharmaceutical composition for use in hair, skin and nail health maintenance. In its optimal embodiment the composition consists of a daily intake of 1 tablet that consists of: Vitamin D3, Vitamin E (natural source), Vitamin C, Thiamin (vitamin B1), Riboflavin (vitamin B2), Niacin (vitamin B3), Vitamin B6, Folic Acid, Vitamin B12, Biotin, Pantothenic Acid, Iron, Magnesium, Zinc, mcg Iodine, Manganese, Copper, Chromium, Selenium, L-Cystine, Natural Mixed Carotenoids, Grape Seed Extract (95% proanthocyanidins); plus 1 Nutri-dermal softgel capsule that consists of: Omega-3 Fish Oil, Lutein, Starflower Oil, Blackcurrant Seed Oil, and Coenzyme Q10.

European Patent No. EP1875897A1 discloses a composition for promoting hair growth that includes fenugreek extract and soy extract in capsule form.

German Published Patent Application No. DE4330597A1 discloses a hair tonic system and hair lotion for hair loss comprising chrysanthemum seeds extracts, rosemary leaf extracts, nettle extracts, fenugreek seeds extract, grape seeds extracts, melon extracts, with other ingredients.

Several inventions in the prior art are already being used to facilitate hair growth and reverse baldness issues in human being, but they either use synthetic compounds in the food supplements or their efficacy is not good enough to give quick results on the hair care, nor they can detoxify the roots of hairs nor facilitate blood circulation around the hair follicles.

Furthermore, even though these innovations may be suitable for the specific purposes to which they address, accordingly, they would not be suitable for the purposes of the present invention as heretofore described. Thus, a solution which can clean the root of hair follicles routinely to revive, stimulate and enhance hair growth and re-growth by activation of hair follicle stem cell by proper detoxification of the root of hair follicle and trigger the blood circulation around the hair follicles by the oral hair care nutritional supplement of the present invention is much needed.

### Detailed Description of the Invention

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary" or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Specific dimensions and other physical characteristics relating to the embodiments disclosed herein are therefore not to be considered as limiting, unless the claims expressly state otherwise.

The present invention now will be described hereinafter with reference to the accompanying examples and/or drawings, in which embodiments of the invention are shown. This description is not intended to be a detailed catalog of all the different ways in which the invention may be implemented, or all the features that may be added to the instant invention. For example, features illustrated with respect to one embodiment may be incorporated into other embodiments, and features illustrated with respect to a particular embodiment may be deleted from that embodiment. Thus, the invention contemplates that in some embodiments of the invention, any feature or combination of features set forth herein can be excluded or omitted. In addition, numerous variations and additions to the various embodiments suggested herein will be apparent to those skilled in the art in light of the instant disclosure, which do not depart from the instant invention. Hence, the following descriptions are intended to illustrate some particular embodiments of the invention, and not to exhaustively specify all permutations, combinations and variations thereof.

Unless otherwise defined, all technical and scientific terms used herein have the same meaning as commonly understood by one of ordinary skill in the art to which this invention belongs. The terminology used in the description of the invention herein is for the purpose of describing particular embodiments only and is not intended to be limiting of the invention. All publications, patent applications, patents and other references cited herein are incorporated by reference in their entireties for the teachings relevant to the sentence and/or paragraph in which the reference is presented.

Unless the context indicates otherwise, it is specifically intended that the various features of the invention described herein can be used in any combination. Moreover, the present invention also contemplates that in some embodiments of the invention, any feature or combination of features set forth herein can be excluded or omitted. To illustrate, if the specification states that a composition comprises components A, B and C, it is specifically intended that any of A, B or C, or a combination thereof, can be omitted and disclaimed singularly or in any combination.

Also as used herein, "and/or" refers to and encompasses any and all possible combinations of one or more of the associated listed items, as well as the lack of combinations when interpreted in the alternative ("or").

One of the main causes of reduction of hair volume is the accumulation of unwanted components in and around the hair follicles. The unwanted components include but are not limited to cellular wastes, metabolites and other contaminants. Any effective formulation of ingredients will be effective in the roots of the hair follicle only when the roots of the hair follicles are complete toxins free and cleaned of all the unwanted components. The solution to above problem is to constantly detoxify and clean the hair follicle sites regularly as an internal process of body physiology by using the chemical free bio based nutritional supplement hair care composition for rejuvenation and proper growth of hair. Hair growth is a continuous cycle process and hair loss is also a natural process, wherein the hair grows from the hair follicle stem cells, falls out and grows again. Hair grows in anagen phase and hair falls out in telogen phase. An optimized way of cyclical regeneration of hair from hair follicle stem cells is required for healthy hair growth. The hair follicle stem cells are responsible for re-generation of hair individually throughout lifetime. The hair follicles in a bald area are generally present in an inactive state and are activated internally as and when the new hair cycle initiates. Each hair follicle works independently for hair re-growth. Regular cleaning of the root of hair follicles and more advantageously everyday cleaning of toxins from root of the hair follicles trigger blood circulation around the hair follicles, thereby facilitates re-generation of hair follicle stem cells. There is always potential chance to re-grow hair in ball head, if hair root are properly cleaned and getting proper blood circulation with essential key nutrients for hair care.

In view of the foregoing, it is therefore a first objective of the present invention to provide a hair care supplement composition that facilitates in detoxification of metabolic wastes and other unwanted substances from the hair root site and to supply the bio optimized and optimum level of all essential nutrients of the hair care supplement composition of the present invention to the hair follicle stem cells of hair bulb to rejuvenate the hair growth.

It is another objective of the present invention to provide a hair care supplement composition that combats hair loss due to a reason including but not limited to the hormonal Dihydrotestosterone (DHT) imbalance that negatively impact the hair regeneration and acts as a DHT blocker in a natural way.

It is another objective of the present invention to provide a hair care supplement composition that elevates oxygen supply and blood circulation. The present supplement composition purifies the blood and increases the oxygen content in the blood. The clean and oxygenated blood enriched with the essential biological ingredients and vitamins reaches to hall areas of head to detoxify the pool of hair follicle stem cells and allows boost of blood oxygen and essential nutrients to the follicles to facilitate hair growth and regeneration.

It is another objective of the present invention to provide a hair care supplement composition that facilitates to generate new neurons around hair follicles.

It is another objective of the present invention to provide a hair care supplement composition that increases the number of Regulatory T cells (Tregs), a type of immune cell generally associated with controlling inflammation, directly trigger stem cells in the skin to promote healthy hair growth.

It is another objective of the present invention to provide a hair care supplement composition that reduces vascular calcification and helps to maintain healthy blood vessels in the hair follicle area.

It is another objective of the present invention to provide a hair care supplement composition that activates potassium channel activity and increase the flow of nutrients into the hair follicle.

It is another objective of the present invention to provide a hair care supplement composition that helps in absorption of all the botanical ingredients into the blood circulation. The present invention aids in active pass of the botanical ingredients from intestine to blood and then to each hair follicle part.

It is another objective of the present invention to provide a hair care supplement composition that cleanses the lymphatic capillaries by delivering key botanical ingredients and vitamins to hair follicle areas and create favorable microenvironment for hair regeneration.

It is another objective of the present invention to provide a hair care supplement composition that promotes proliferation of Dermal Papilla Cells (DPC) that plays a key role in hair follicle development and hair growth and inhibit 5α-reductase that instigates DHT production.

It is another objective of the present invention to provide a hair care supplement composition that balances pH around hair root areas, fights against dandruff internally and improves the appearance of the hair, making it look shinier and healthier.

It is another objective of the present invention to provide a hair care supplement composition that neutralizes free radical damage. The botanical ingredients in the present invention provide antioxidants that help to get rid of hair damaging compounds.

It is another objective of the present invention to provide a hair care supplement composition that helps in retaining moisture in the hair follicles and produce phospholipids which make up the hair follicle membranes.

It is another objective of the present invention to provide a hair care supplement composition that has an anti-ageing effect on hair health, as it boosts the process of gene expression which is involved in the synthesis of keratin.

It is another objective of the present invention to provide a hair care supplement composition that increases the size, depth and length of hair follicle for better growth of hair.

According to a first aspect, to achieve the above enumerated goals, the invention provides a hair care oral nutritional supplement composition, comprising:
- fenugreek seed extract;
- ashwagandha root extract;
- bambusa bamboo extract;
- moringa seed extract.

As used herein, the term "nutritional supplement" can be understood as and used interchangeably with the terms "food supplement", "nutraceutical composition" and/or "dietary supplement".

As used herein, the term "oral" means that the composition according to the invention is taken through the mouth. This includes buccal, sublabial and sublingual administration.

According to an embodiment of the present invention, the present invention provides a hair care supplement composition that not only improves cyclical regeneration of hair from hair follicle stem cells within human body but also works against accumulation of unwanted components in and around the hair follicles. The present supplement composition comprises key ingredients that works synergistically to activate the hair follicles by proper detoxification of the root of hair follicle and trigger the blood circulation around the hair follicles.

According to another embodiment of the present invention the supplement composition comprises several selected vitamins, minerals, selected plant extracts, proteins etc in ratio to act as a botanical hair care supplement composition, wherein the hair care supplement composition acts as a hair progenitor, thereby facilitating to provide increases the oxygen supply and blood flow with essential nutrients delivery to hair follicle area by opening potassium channels to rejuvenate hair.

In a preferred embodiment, the hair care oral nutritional supplement composition further comprises:
- vitamin C;
- vitamin K2;
- zinc.

In a particular embodiment, the hair care oral nutritional supplement composition according to the invention comprises:
- fenugreek seed extract in a range of 1.5 % to 7.8 % w/w;
- Ashwagandha root extract in a range of 1.5 % to 7.5 % w/w;
- bambusa bamboo extract in a range of 5.5 % to 11% w/w;
- moringa seed extract in a range of 3.5% to 10.5% w/w;
- vitamin C in a range of 7% to 13% w/w;
- vitamin K2 in a range of 0,28 % to 2 % w/w;
- zinc in a range of 3 % to 7 % w/w.

In a particular embodiment, the hair care oral nutritional supplement composition according to the invention further comprises at least one, preferably all, of:
- saw palmetto berries extract;
- nettle root extract;
- chrysanthellum indicum flower extract;
- rosemary leaf extract;
- grape seed extract;
- millet seed extract;
- inositol;
- super oxyde dismutase melon.

In a particular embodiment, the hair care oral nutritional supplement according to the invention comprises:
- saw palmetto berries extract in a range of 3.2% to 8.5% w/w;
- nettle root extract in a range of 1.3% to 5.6% w/w;
- chrysanthellum indicum flower extract in a range of 4.5% to 9.5% w/w;

- rosemary leaf extract in a range of 3.8% to 8.9% w/w;
- grape seed extract in a range of 3.1% to 7.2% % w/w;
- millet seed extract in a range of 2.5% to 8.2% w/w;
- inositol in a range of 0.7% to 3.5% w/w;
- super oxyde dismutase melon in a range of 0.3% to 2% w/w.

In a particular embodiment, the hair care oral nutritional supplement according to the invention further comprises at least one of: eucalyptus extract, pumpkin seed extract, green tea extract, licorice, reishi mushroom extract, eckolonia cava extract, tomato extract, or combination thereof.

In a particular embodiment, the hair care oral nutritional supplement according to the invention further comprises Lactoferrin.

According to another exemplary embodiment of the present invention the hair care supplement composition comprises Eckolonia Cava extract, Saw palmetto berries extract, Reishi Mushroom extract, Fenugreek seed Extract, Pumpkin seed extract, green tea (C sinensis) extract, Rosemary (Rosmarinus officinalis) extract, Grape seed (Vitis vinifera) extract, Licorice (G glabra) and Tomato extract.

In a particular embodiment, the hair care oral nutritional supplement according to the invention comprises botanicals extracts in a range of 15% to 50% w/w of the total weight, preferably wherein the botanical extracts are selected from at least one of: eckolonia cava extract, reishi mushroom extract, pumpkin seed extract, green tea extract, licorice, tomato extract, vitamin K2, vitamin C, lactoferrin, saw palmetto berries extract, fenugreek seed extract, nettle root extract, ashwagandha root extract, chrysanthellum indicum flower extract, rosemary leaf extract, grape seed extract, millet seed extracts, moringa seed extract, or combination thereof.

According to another exemplary embodiment of the present invention the hair care nutritonal supplement composition comprises Eckolonia Cava extract, Saw palmetto berries extract, Reishi Mushroom extract, Fenugreek seed Extract, Pumpkin seed extract, Green tea (C sinensis) extract, Rosemary (Rosmarinus officinalis) extract, Grape seed (Vitis vinifera) extract, Licorice (G glabra), Tomato extract, Vitamin K2, Ashwagandha Root extract, Vitamin C, Lactoferrin, Moringa seed extract, Millet (Panicum miliaceum) extract, Bambusa bamboo extract, Nettle root extract, Chrysanthellum indicum flower extract, Inositol, SOD Melon extract, Coenzyme Q10 (Ubiquinol), and Eucalyptus extract.

According to another exemplary embodiment of the present invention the hair care nutritional supplement composition comprises Eckolonia Cava extract, Reishi Mushroom extract, Pumpkin seed extract, Green tea (C sinensis) extract, Licorice (G glabra), Tomato extract, Vitamin K2, Vitamin C extract, Lacttoferrin, Saw palmetto berries extract, Fenugreek seed extract, nettle root extract (Urtica dioica), Ashwagandha root extract, Chrysanthellum indicum flower extract, Rosemary leaf extract (ursolic acid), Grape seed (Vitis vinifera OPC) extract, Millet seed extracts (Panicum miliaceum L.), Moringa seed extract (Moringa oleifera Lam), Giant Bambou (Bambusa bamboo) extract, Vitamin C, Inositol, SOD Melon (super oxyde dismutase ) extract, Co-enzyme 10, L- Arginine, L-Tyrosine, L Lysine HCl, Vitamin K2, Vitamin D3,Vitamin B1 (Thiamin ), Vitamin B2 (Riboflavine), Vitamin B3 (Nicotinamide ), Vitamin B5 (Calcium D - Pantothenate), Vitamin B6 (Pyridoxine), Vitamin B8 (Biotin), Vitamin B9 (A.Folique), Vitamin B12 (Cyanocobalamine), Zinc (Zn bisglycinate), Gluconate Cuivre, Molybdate de Sodium, selenium - L selenomethionine, and banana flour as ecoulement agent.

According to another exemplary embodiment of the present invention, a premix of biological ingredients of the hair care supplement composition comprises botanicals extracts selected from Eckolonia Cava extract, Reishi Mushroom extract, Pumpkin seed extract, Green tea (C sinensis) extract, Licorice (G glabra), Tomato extract, Vitamin K2, Vitamin C, Lacttoferrin , Saw palmetto berries extract, Fenugreek seed extract (graines Fenugrec), nettle root extract (Urtica dioica), ashwagandha root extract, Chrysanthellum indicum flower extract (Camomille d'or, Chamomile) Rosemary leaf extract (ursolic acid), Grape seed (Vitis vinifera OPC) extract, Millet seed extracts (Panicum miliaceum L.), Moringa seed extract (Moringa oleifera Lam), Giant Bamboo extract (Bambusa bambos L.) and a combination thereof in a range of 15% to 50% w/w of the total weight of the hair care supplement composition.

In another exemplary embodiment of the present invention a hair care nutritional supplement composition, wherein the composition comprises botanicals extracts in a range of 15% to 50% w/w of the total weight (100% w/w) of the hair care supplement composition, wherein the botanical extract is selected from eckolonia cava extract, reishi mushroom extract, pumpkin seed extract, green tea extract, Licorice, Tomato extract, vitamin K2, Vitamin C, Lactoferrin, saw palmetto berries extract, Fenugreek seed extract, nettle root extract, ashwagandha root extract, chrysanthellum indicum flower extract, rosemary leaf extract, grape seed extract, millet seed extracts, Moringa seed extract, giant bamboo extract and a combination thereof.

In a particular embodiment, the hair care oral nutritional supplement according to the invention further comprises L-Cysteine and/or choline.

In a particular embodiment, the hair care oral nutritional supplement according to the invention further comprises at least one of: co-enzyme 10, L- arginine, L-tyrosine, L-lysine HCl, vitamin D3, vitamin B1, vitamin B2, vitamin B3, vitamin B5, vitamin B6, vitamin B8, vitamin B9, vitamin B12, copper gluconate, sodium molybdate, selenium, banana flour or combination thereof.

In a particular embodiment, the hair care oral nutritional supplement according to the invention comprises:
- 5.88% w/w saw palmetto berries extract;
- 2.35% w/w fenugreek seed extract;
- 2.35% w/w nettle root extract;
- 2.35% w/w ashwagandha root extract;
- 5.88% w/w chrysanthellum indicum flower extract;
- 5.88% w/w rosemary leaf extract;
- 4.705% w/w grape seed extract;
- 4.705% w/w millet seed extract;
- 4.705% w/w moringa seed extract;
- 7.06% w/w bambusa bamboo extract;
- 8.26% w/w vitamin C ;
- 1.18% w/w inositol;
- 0.78% w/w super oxyde dismutase melon;
- 11.76% w/w co-enzyme 10;
- 11.76% w/w L- arginine;
- 2.35% w/w L-tyrosine;
- 2.35% w/w L-lysine HCl;
- 0.65% w/w vitamin K2;
- 0.24% w/w vitamin D3 ;
- 0.09% w/w vitamin B1;
- 0.12% w/w vitamin B2;
- 1.38% w/w vitamin B3;
- 0.52% w/w vitamin B5;
- 0.12% w/w vitamin B6;
- 0.005% w/w vitamin B8;
- 0.02% w/w vitamin B9;
- 0.0002% w/w vitamin B12;
- 3.92% w/w zinc;
- 0.12% w/w copper gluconate;
- 0.59% w/w sodium molybdate;
- 0.86% w/w selenium;
- 7.06% w/w banana flour.

In a particular embodiment, the hair care oral nutritional supplement is in a form selected from the group of: a tablet, a capsule, a candy, a pouch, a food supplement package, a toffee, a bar, a lozenz, sauce, juice or a combination thereof.

According to the invention, "tablet" means an oral solid dosage form. It comprises a mixture of active substances and excipients, usually in powder form, pressed or compacted from a powder into a solid dose. Tablets are prepared either by molding or by compression. Tablets comprises in particular the forms of Pills, caplets, and orally disintegrating tablets.

According to the invention, "capsule" refers to the encapsulation of dosage forms, in particular in the way of hard-shelled capsules and soft-shelled capsules. Hard-shelled contain dry, powdered ingredients or miniature pellets made by e.g. processes of extrusion or spheronization. These are made in two halves: a smaller-diameter "body" that is filled and then sealed using a larger-diameter "cap". Soft-shelled capsules are primarily used for oils and for active ingredients that are dissolved or suspended in oil.

According to the invention, "lozenz" means a small chocolate form of candy.

According to the invention, "candy" means a form similar to a sweet food made from sugar or chocolate.

According to the invention, "toffee" means a chewy or crunchy form made with brown sugar or molasses and butter, often coated with nuts.

According to the invention, "pouch" means a sealed plastic or foil container used for packaging food.

According to the invention, "food supplement package" means any container used for packaging food supplement, in particular jars, bottles, cans, flasks, tanks, etc.

According to the invention, "bar" means a food preparation easily transportable and often rich in carbohydrates and proteins, having often the form of rectangular parallelepiped.

According to the invention, "juice" means that the food supplement according to the invention is introduce before its conditioning into juice, in particular vegetable or fruit juice.

According to the invention, "sauce" means a liquid form where the food supplement according to the invention is introduce before its conditioning, in particular composed at least in part of vegetable or meat, and use to give tast of food products.

In a particular embodiment, the hair care oral nutritional supplement according to the invention is in a form of a capsule, wherein the capsule comprises a capsule cover made of nano particles of gold or copper or a combination thereof with other bonding agents.

The hair-follicle stem cells send message to surrounding lymphatic capillaries to remove accumulated unwanted skin cells, toxic components as well as to remove excess fluids for regeneration of hair from the hair follicle, thus facilitating regeneration of hair. Lymphatic capillaries are more so often inflamed near the hair follicle thus regular drainage to messy things of hair areas is required to get new hair along with healthy skin. The composition of the present invention provides a combination of botanical DTH blockers, which can clean the messy and unwanted cell, fluid etc. in very efficient way. In fact, the molecules of the composition of the present invention not only clean the messy things of lymphatic capillaries but also create all favorable microenvironments for hair re-growth. Hair loss is also related to impaired lymphatic capillaries (not able to drain the fluid, toxins, unhealthy hair stem cells etc.).

Moringa seed extract (Moringa oleifera Lam) of the hair care supplement composition comprises key amino acids and peptides, further it is rich in phytosterols like stigmasterol, sitosterol and kampesterol which are precursors for various hormones. Moringa further comprises vitamin C, vitamin A, calcium, and potassium. Moringa in presence of all the botanical ingredients create efficient RBC in our blood. It makes hormonal balance and pH balance in the blood.

Preferably, Moringa seed extract is present in the composition according to the invention in a range of 3.5% to 10.5% w/w.

Bambusa bamboo (Bambusa bambos (L.)) as an ingredient of the composition on reaching the intestine gets digested by the friendly gut micro-biota to secret pluralities of hair regenerative factors, thereby facilitating hair care naturally. Furthermore, Bambusa bamboo is a good source of natural silica, a nutrient that is integral in the growth of hair, Bamboo extract contains over 70% organic silica and is 10 times more potent than horsetail silica and further it produces 35% more Oxygen than other plants, further Bambusa bamboo has an antioxidant property that helps detoxify our body and also facilitate in better nutrient absorption.

Preferably, Bambusa bamboo extracts is present in the composition according to the invention in a range of 5.5 % to 11% w/w.

Fenugreek seed extract of the hair care supplement composition activates the stem cells in the hair follicle to make hair grow by balancing pH around hair root areas and is also effective against dandruff by internally acting against it.

In a preferred embodiment the fenugreek seed extract is present in a range of 1.5% to 7.8% w/w of the total weight of the hair care supplement composition.

Ashwagandha root extract of the hair care supplement composition comprises whitanolides. It is useful to generate new neurons around hair follicles. Ashwagandha root extract creates functional neurons that are necessary for all type of cell regeneration. It further contains high levels of antioxidants to protect hair from free radicals. The composition of the present invention facilitates to create new blood vessels so called neovascularization and new neurons. Those newly created thin blood vessels are very efficient to ample amount of blood flow to hair follicles stem cells areas.

In a preferred embodiment the Ashwagandha root extract comprises in a range of 1.5% to 7.5% w/w of the total weight of the hair care supplement composition.

Optimized dose of Vitamin C speeds up the movement of all ingredients from intestinal absorption to blood circulation and finally deliver to hair root areas where hair follicle stem cells are present.

In a preferred embodiment, the vitamin C is in a range of 7% to 13% w/w of the total weight of the hair care supplement composition.

Vitamin K2 Decalcify the Scalp. Calcification of blood vessels can happen anywhere in the body, including the scalp. Scalp calcification is directly linked to hair loss. It happens when the blood vessels underneath the scalp accumulate calcium in their walls. Vascular calcification decreases nutrient delivery which means less of the building blocks and co-factors required to initiate hair growth. So, maintaining healthy blood vessels by reducing calcification is important for supporting hair growth. Calcification of blood vessels in the scalp reduces blood flow, thereby, diminishes nutrient delivery to the follicle required for hair growth. Thus, maintaining healthy blood vessels by reducing calcification is important for supporting hair growth.

In a preferred embodiment of the present invention, the hair care supplement composition comprises vitamin K2 in a range of 0,28 % to 2 % of the total weight of the hair care supplement composition.

Zinc (Bisglycinate or glycinate) acts as an anti-inflammatory agent and supports hair regeneration.

According to another preferred embodiment of the present invention, the hair care supplement composition comprises zinc in a range of 3 % to 7 % w/w of the total weight of the hair care supplement composition.

Saw palmetto berries extract has antiandrogenic properties and inhibit an enzyme called 5-alpha-reductase, that converts testosterone to DHT, and lowers in this way the levels of DHT. DHT is known to be the molecule responsible for hair loss.

Preferably, saw palmetto berries extract in the present invention is present in a range of 3.2% to 8.5% w/w of the total weight of the hair care supplement composition.

Ortie racine extract (nettle root, Urtica dioica) of the hair care supplement composition neutralizes free radical damage and blocks DHT topically and internally enhances blood circulation, so that hair roots get more supply of nutrients and oxygen. It has super antioxidants that help to get rid of hair damaging compounds

In a preferred embodiment the nettle root extract (Urtica dioica) is in a range of 1.3% to 5.6% w/w of the total weight of the hair care supplement composition.

According to another exemplary embodiment of the present invention Chrysanthellum indicum of the botanical hair care supplement composition activates hair growth activity and hair pigmentation.

In a preferred embodiment the Chrysanthellum indicum is in a range of 4.5% to 9.5% w/w of the total weight of the hair care supplement composition.

According to another exemplary embodiment of the present invention rosemary oil contains ursolic acid which helps to increase hair follicle blood circulation and promotes healthy hair growth. Rosemary leaf extract also repairs the damaged nerves in the scalp and functional nerves in the scalp. It minimises the effect of testosterone. Rosemary oil further comprises about 22 unique components, with the most significant natural compounds cineole and α-pinene. These components are effective against dandruff formation due to its antifungal and antimicrobial property.

In a preferred embodiment the rosemary oil comprises in a range of 3.8% to 8.9% w/w of the total weight of the hair care supplement composition.

In an exemplary embodiment the hair care supplement composition of the present invention grape seed of the hair care supplement composition comprises proanthocyanins that helps in the proliferation of hair follicle cells stimulates hair cell production and speed up hair growth.

In a preferred embodiment the grape seed is in a range of 3.1% to 7.2% w/w of the total weight of the hair care supplement composition.

Millet seed extract increase the blood supply to hair follicles allowing boost of blood, oxygen, and essential nutrients to the follicles which helps hair physiological cycle. L-Cysteine and Millet Seed Extract and Pantothenic Acid (Vitamin B5) facilitate in reversing hair loss.

According to another preferred embodiment of the present invention, the hair care supplement composition comprises Millet (Panicum miliaceum) seed extract in a range of 2.5% to 8.2% w/w of the total weight of the hair care supplement composition.

Inositol strengthens each follicle so that it retains moisture. This is especially great, given that one of the main issues with growing long, natural hair is that it often doesn't retain enough moisture. This often causes breakage amongst follicles as the hair can turn brittle without the ability to remain properly moisturized. This makes inositol one of essential for hair growth.

According to another preferred embodiment of the present invention, the hair care supplement composition comprises inositol in a range of 0.7% to 3.5% w/w of the total weight of the hair care supplement composition.

According to another preferred embodiment of the present invention, although inositol works great on its own, it is said to be even powerful when mixed with choline. Choline is a strong and basic, water-soluble compound that is naturally occurring in living tissues. When these inositol and choline are combined, they produce phospholipids which make up the hair follicle membranes. This provides added protection for hair follicles overall.

According to another preferred embodiment of the present invention, the hair care supplement composition comprises choline in a range of 2% to 4.6% w/w of the total weight of the hair care supplement composition.

Watermelon rind or super oxyde dismutase melon (SOD Melon) extract stimulates the secretion of nitric oxide of L-arginine and L-citrulline and helps in absorption of nutrients in the intestine. Further Watermelon rind extract increase the cellular (hair cells and blood circulation) level of superoxide dismutase (SOD). It is more powerful than antioxidant vitamin as it activates natural production of antioxidant inside human body including catalase and glutathione peroxidase.

According to another preferred embodiment of the present invention, the hair care supplement composition comprises super oxyde dismutase melon in a range of 0.3% to 2% w/w of the total weight of the hair care supplement composition.

The present invention may employ Eucalyptus extract that reduces the inflammation to create ideal environment for hair growth. The eucalyptus oil in the present invention is used in the pre-determined concentration.

According to another preferred embodiment of the present invention, the hair care supplement composition comprises Eucalyptus extract in a range of 0.5% to 1.5% w/w of the total weight of the hair care supplement composition.

According to another exemplary embodiment of the present invention the hair care supplement composition additionally comprises quantity of Reishi Mushroom extract, Pumpkin seed extract, green tea (C sinensis) extract, licorice eckolonia cava extract, tomato extract, or combination thereof.

According to another preferred embodiment of the present invention, the hair care supplement composition comprises Reishi mushroom extract in a range of 2.5% to 7.2% w/w of the total weight of the hair care supplement composition.

According to another preferred embodiment of the present invention, the hair care supplement composition comprises Pumkin seed extract in a range of 1.2% to 3.5% w/w of the total weight of the hair care supplement composition.

According to another preferred embodiment of the present invention, the hair care supplement composition comprises green tea extract in a range of 1.7% to 2.6% w/w of the total weight of the hair care supplement composition.

According to another preferred embodiment of the present invention, the hair care supplement composition comprises licorice eckolonia cava extract in a range of 2.3% to 6.3% w/w of the total weight of the hair care supplement composition.

According to another preferred embodiment of the present invention, the hair care supplement composition comprises tomato extract extract in a range of 2.1% to 3.6% w/w of the total weight of the hair care supplement composition.

According to another exemplary embodiment of the present invention Eckolonia cava extract of the hair care supplement composition proliferates dermal papilla cells (DPC) that plays a key role in hair follicle development and hair growth. Further the Eckolonia cava extract inhibits 5α-reductase activity which is a known instigator of DHT production activity. Enzymes of E. Cava can induce anagen phase in mice, promoting proliferation of Dermal Papilla Cells (DPC) and possibly even inhibiting 5α-reductase (the instigator of DHT production) activity that promote human hair growth via the proliferation of hDPCs with ROS scavenging and increasing growth factors such as IGF-1 and VEGF. Furthermore, the Eckolonia cava extract mediates the development of blood vessels. With proper blood flow, hair growth rates are boosted and follicle and hair thickness is increased.

According to another exemplary embodiment of the present invention, Lactoferrin acts as a multitasking natural protein, effectively regulates the body's iron levels, promotes the growth of "good" bacteria for optimal gut health and stimulate Nitric oxide secretion on cells so as to improve oxygen and nutrient delivery to every cell in the body, including the cells of the hair follicles. One example of lactoferrin is the one registered under proferrin.

According to another preferred embodiment of the present invention, the hair care supplement composition comprises lactoferrin in a range of 2% to 8% w/w of the total weight of the hair care supplement composition.

According to another preferred embodiment of the present invention, the hair care supplement composition additionally comprises L-Cysteine, preferably in a range of 0.5% to 1 % w/w of the total weight of the hair care supplement composition.

According to another preferred embodiment of the present invention, the hair care supplement composition comprises L-Arginine, preferably in a range of 7% to 15% w/w of the total weight of the hair care supplement composition.

According to another preferred embodiment of the present invention, the hair care supplement composition comprises L-Tyrosine, preferably in a range of 1.5% to 7.5% w/w of the total weight of the hair care supplement composition.

According to another preferred embodiment of the present invention, the hair care supplement composition comprises L-Lysine, preferably in a range of 1.5% to 7.5% w/w of the total weight of the hair care supplement composition.

Coenzyme Q10 (Ubiquinol) has an anti-ageing effect on hair health, as it boosts the process of gene expression which is involved in the synthesis of keratin. According to another preferred embodiment of the present invention, the hair care supplement composition comprises Coenzyme Q10 (Ubiquinol) in a range of 7% to 15% w/w of the total weight of the hair care supplement composition.

According to another preferred embodiment of the present invention, the hair care supplement composition comprises 0.09% w/w Vitamin B1 (Thiamin) 100% VNR, 0.12% w/w Vitamin B2 (Riboflavine) 100% VNR, 1.38% w/w Vitamin B3 (Nicotinamide) 100% VNR, 0.52% w/w Vitamin B5 (Calcium D - Pantothenate) 100% VNR, 0.12% w/w Vitamin B6 (Pyridoxine) 100% VNR, 0.005% w/w Vitamin B8 (Biotin) 100% VNR, 0.02% w/w Vitamin B9 (A.Folique) 100% VNR, 0.0002% w/w Vitamin B12 (Cyanocobalamine) 100% VNR

"100% VNR" means according to the European food safety authority that the totality of the "Nutrient Reference Values" are comprises regarding the named component for one day for an average adult. VNR are a set of numerical food values that cover: the average requirement (AR), the population nutrient reference (PNR), the Adequate Intake (AI), and the macronutrient reference range (IR). These values provide professional guidance on the amounts of nutrients needed to maintain a balanced diet for an otherwise healthy individual or group of individuals. NRVs also cover the upper safe limit (USL), which is the maximum amount of a nutrient that can be safely consumed over a long period of time.

According to another preferred embodiment of the present invention, the hair care supplement composition comprises Sodium Molybdate, preferably in a range of 0.25% to 2% w/w of the total weight of the hair care supplement composition.

According to another preferred embodiment of the present invention, the hair care supplement composition comprises Gluconate copper, preferably in a range of 0.08% to 0.5% w/w of the total weight of the hair care supplement composition.

According to another preferred embodiment of the present invention, the hair care supplement composition comprises vitamin D3, preferably in a range of 0.10% to 0.5% w/w of the total weight of the hair care supplement composition, wherein vitamin D3 helps destroy free radicals.

According to another preferred embodiment of the present invention, the hair care supplement composition comprises selenium (L-Selenomethionine) which provides serious hair protection from UV damage by increasing the levels of selenium-dependent antioxidant proteins prior to UV exposure.

The L-Selenomethionine is added in the composition of the present invention in a range of 0.3% to 1.5% w/w of the total weight of the hair care supplement composition.

The hair care supplement composition of the present invention contains ingredients that work parallelly and synergistically to speed up the movement of all ingredients of the composition as well nutrients of our daily food to hair follicle pool site, i.e. hair root.

In another exemplary embodiment of the present invention the hair care nutritional supplement composition in the form of a capsule comprising a capsule cover made of nano particle of gold or copper or a combination thereof in other bonding agents like gelatin or the like, wherein nano particles of gold and copper facilitate in hair growth.

The hair care formula is deeply focus to detoxify, clean the root of hair follicle while delivering all formulated ingredients to the root of hair (hair follicle stem cells pool), just like proper food for hair follicle stem cells. The hair care nutritional supplement in the form of a capsule is evaluated in vitro using human hair follicle stem cells in organoid model and tested in various pilot and randomized trials. Results are very impressive, especially prevention of hair loss and excellent for hair care. Daily consumption of the capsule results in hair loss prevention, improve the hair re-growth. The composition increases blood flow and bring clean blood by to hall areas of head by reducing the inflammation of blood and successfully bring those essential required ingredients to the pool of hair follicle stem cells. Furthermore, the key ingredients of formula generate new neurons in head areas.

According to another aspect of the present invention, the hair care supplement composition of the present invention activates hair follicle stem cells internally along with cellular and molecular hair regenerative factors to prevent hair loss while stimulation of hair re-growth.

According to an aspect of the present invention, the hair care nutritional supplement composition kick start the trigger for hair regrowth along with hair prevention by using 100% drug free optimize dose of botanical extracts.

According to another aspect of the present invention, the hair care supplement composition effectively detoxifies each hair root and inhibits accumulation of undesirable components including but not limited to cellular wastes, metabolites and other contaminants in the root of hair that dysfunctions natural power of the hair follicle stem cells for generation of new hair.

According to a second aspect, the invention provides a use of a hair care oral nutritional supplement according to the invention for the non-therapeutic prevention and/or reduction of hair loss, thinning hair problems, balding disorders and regeneration of hairs in a natural way.

According to a third aspect, the invention provides a hair care oral nutritional supplement according to the invention for use in the prevention and/or reduction of hair loss, thinning hair problems, balding disorders and regeneration of hairs in a natural way.

By "natural way" is meaning according to the invention that there is no artificial implementation of hairs, and that the regeneration of hairs are made by the body itself.

According to a preferred embodiment of the invention, the composition is taken at least once a day.

According to a more preferred embodiment of the invention, the composition is taken 3 or 4 times per day.

The purpose of this multiple intakes per day is correlated with the root of the hair being constantly contaminated with our own cellular wastes from our body and outside. It is important to detoxify and clean the root of each hair every 5 to 8 hours.

### Figures

Figure 1: This figure represents 5 photographs of individual number 1, taken from all angles (front of the head, profiles, back of the head, top of the head), before taking the hair care nutritional supplement composition according to the invention.
Figure 2: This figure represents 5 photographs of individual number 1, taken from all angles (front of the head, profiles, back of the head, top of the head), after 12 weeks of taking the hair care nutritional supplement composition according to the invention.
Figure 3: This figure represents 5 photographs of individual number 2, taken from all angles (front of the head, profiles, back of the head, top of the head), before taking the hair care nutritional supplement composition according to the invention.
Figure 4: This figure represents 5 photographs of individual number 2, taken from all angles (front of the head, profiles, back of the head, top of the head), after 12 weeks of taking the hair care nutritional supplement composition according to the invention.
Figure 5: This figure represents 5 photographs of individual number 3, taken from all angles (front of the head, profiles, back of the head, top of the head), before taking the hair care nutritional supplement composition according to the invention.
Figure 6: This figure represents 5 photographs of individual number 3, taken from all angles (front of the head, profiles, back of the head, top of the head), after 12 weeks of taking the hair care nutritional supplement composition according to the invention.
Figure 7: This figure represents 5 photographs of individual number 4, taken from all angles (front of the head, profiles, back of the head, top of the head), before taking the hair care nutritional supplement composition according to the invention.
Figure 8: This figure represents 5 photographs of individual number 4, taken from all angles (front of the head, profiles, back of the head, top of the head), after 12 weeks of taking the hair care nutritional supplement composition according to the invention.
Figure 9: This figure represents 5 photographs of individual number 5, taken from all angles (front of the head, profiles, back of the head, top of the head), before taking the hair care nutritional supplement composition according to the invention.
Figure 10: This figure represents 5 photographs of individual number 5, taken from all angles (front of the head, profiles, back of the head, top of the head), after 12 weeks of taking the hair care nutritional supplement composition according to the invention.
Figure 11: This figure represents 5 photographs of individual number 6, taken from all angles (front of the head, profiles, back of the head, top of the head), before taking the hair care nutritional supplement composition according to the invention.
Figure 12: This figure represents 5 photographs of individual number 6, taken from all angles (front of the head, profiles, back of the head, top of the head), after 12 weeks of taking the hair care nutritional supplement composition according to the invention.
Figure 13: This figure represents 5 photographs of individual number 7, taken from all angles (front of the head, profiles, back of the head, top of the head), before taking the hair care nutritional supplement composition according to the invention.
Figure 14: This figure represents 5 photographs of individual number 7, taken from all angles (front of the head, profiles, back of the head, top of the head), after 12 weeks of taking the hair care nutritional supplement composition according to the invention.
Figure 15: This figure represents 5 photographs of individual number 8, taken from all angles (front of the head, profiles, back of the head, top of the head), before taking the hair care nutritional supplement composition according to the invention.
Figure 16: This figure represents 5 photographs of individual number 8, taken from all angles (front of the head, profiles, back of the head, top of the head), after 12 weeks of taking the hair care nutritional supplement composition according to the invention.
Figure 17: This figure represents 5 photographs of individual number 9, taken from all angles (front of the head, profiles, back of the head, top of the head), before taking the hair care nutritional supplement composition according to the invention.
Figure 18: This figure represents 5 photographs of individual number 9, taken from all angles (front of the head, profiles, back of the head, top of the head), after 12 weeks of taking the hair care nutritional supplement composition according to the invention.
Figure 19: This figure represents 5 photographs of individual number 10, taken from all angles (front of the head, profiles, back of the head, top of the head), before taking the hair care nutritional supplement composition according to the invention.
Figure 20: This figure represents 5 photographs of individual number 10, taken from all angles (front of the head, profiles, back of the head, top of the head), after 12 weeks of taking the hair care nutritional supplement composition according to the invention.

### Examples

The invention will now be described with reference to the following examples. It should be appreciated that these examples are not intended to limit the scope of the claims to the invention, but are rather intended to be exemplary of certain embodiments. Any variations in the exemplified methods that occur to the skilled artisan are intended to fall within the scope of the invention.

### EXAMPLE 1

**Table: 1 (Exemplary hair care nutritional supplement composition 1)**

| Ingredients | Percentage Range (w/w) |
|---|---|
| Fenugreek seed ES | 1.5 % to 7.8 % |
| Ashwagandha ES BIO | 1.5% to 7.5 % |
| *Bambusa bamboo* | *5.5* % *to 11%* |
| Moringa seed ES Bio *Moringa oleifera Lam* | *3.5% to 10.5%* |
| Vitamin C 100% VNR 97.5% enrobé | 7 % to 13% |
| Vitamin K2 | 0.28% to 2% |
| Zinc | 3% to 7% |

Table 1 shows a hair care nutritional supplement composition, wherein the composition comprises: fenugreek seed extract in a range of 1.5 % to 7.8 % w/w; Ashwagandha root extract in a range of 1.5 % to 7.5 % w/w; bambusa bamboo extract in a range of 5.5 % to 11% w/w; moringa seed extract in a range of 3.5% to 10.5% w/w; vitamin C in a range of 7% to 13%w/w; vitamin K2 in a range of 0,28 % to 2 % w/w; and zinc in a range of 3 % to 7 % w/w.

### EXAMPLE 2

**Table: 2 (Exemplary hair care nutritional supplement composition 2)**

| Ingredients | Percentage Range (w/w) |
|---|---|
| Fenugreek seed ES | 1.5 % to 7.8 % |
| Ashwagandha ES BIO | 1.5% to 7.5 % |
| *Bambusa bamboo* | *5.5 % to 11%*% |
| Moringa seed ES Bio *Moringa oleifera Lam* | *3.5% to 10.5%* |
| Vitamin C 100% VNR 97.5% enrobé | 7 % to 13% |
| Vitamin K2 | 0.28% to 2% |
| Zinc | 3% to 7% |
| Saw palmetto ES | 3.2 to 8.5 % |
| Ortie racine ES (nettle root, *Urtica dioica)* | 1.3 % to 5.6% |
| *Chrysanthellum indicum* ES | 4.5 % to 9.5 % |
| Rosemary ES (ursolic acid) Romarin | 3.8 % to 8.9% |
| Grape seed (*Vitis vinifera* OPC) | 3.1 % to 7.2 % |
| *Millet ES (47% methionine) Panicum miliaceum L.* | *2.5% to 8.2%* |
| Inositol | 0,7 % to 3 % |
| SOD Melon (*super oxyde dismutase)* | 0.3% to 2% |

Table 2 shows a hair care nutritional supplement composition, wherein the composition comprises: fenugreek seed extract in a range of 1.5 % to 7.8 % w/w; Ashwagandha root extract in a range of 1.5 % to 7.5 % w/w; bambusa bamboo extract in a range of 5.5 % to 11% w/w; moringa seed extract in a range of 3.5% to 10.5% w/w; vitamin C in a range of 7% to 13% w/w; vitamin K2 in a range of 0,28 % to 2 % w/w; zinc in a range of 3 % to 7 % w/w; saw palmetto berries extract in a range of 3.2% to 8.5% w/w; nettle root extract in a range of 1.3% to 5.6% w/w; chrysanthellum indicum flower extract in a range of 4.5% to 9.5% % w/w; rosemary leaf extract in a range of 3.8% to 8.9% w/w; grape seed in a range of 3.1% to 7.2% w/w; millet seed extract in a range of 2.5% to 8.2% w/w; inositol in a range of 0.7% to 3.5% w/w; and SOD melon in a range of 1.5 % to 7.5 % w/w.

The above tables 1 and 2 show exemplary hair care nutritional supplement composition as a oral formulation either as a tablet, a capsule, a candy, a pouch, a stick a food supplement package, a toffee, a bar, a lozenz, a semisolid form such as sauce, liquid form such as juice or the like or a combination thereof, which can be mixed with any drinks or food or taken independently as a nutritional supplement. The approximative size of the oral supplement is convenient as a single serving size that is perfect for no-fuss preparation at home or on the go.

### EXAMPLE 3

**Table: 3 (Exemplary hair care nutritional supplement composition 3)**

| Ingredients | mg (wt) | Percentage (w/w) |
|---|---|---|
| Saw palmetto berries extract | 25.00 | 5.88% |
| Fenugreek seed *extract* | 10.00 | 2.35% |
| Ortieracine (nettleroot, *Urticadioica) extract* | 10.00 | 2.35% |
| Ashwagandha root extract | 10.00 | 2.35% |
| *Chrysanthellum indicum flower extract* | 25.00 | 5.88% |
| Rosemary (ursolicacid) Romarin *extract* | 25.00 | 5.88% |
| Grape seed (*Vitis vinifera*OPC) *extract* | 20.00 | 4.705% |
| Millet (methionine) *Panicum miliaceum extract* | 20.00 | 4.705% |
| Moringa seed *extract* | 20.00 | 4.705% |
| Giant bamboo/*Bambusa bamboo extract* | 30.00 | 7.06% |
| Vitamin C 100% VNR 97.5% | 35.10 | 8.26% |
| Inositol | 5.00 | 1.18% |
| SOD Melon (*super oxyde dismutase*) *extract* | 3.30 | 0.78% |
| Co-enzyme 10 | 50.00 | 11.76% |
| L- Arginine | 50.00 | 11.76% |
| L-Tyrosin | 10.00 | 2.35% |
| L LysineHCl | 10.00 | 2.35% |
| Vitamin K2 10000 ppm 100% VNR (75µg) | 2.750 | 0.65% |
| Vitamin D3 (0,25%) 100% VNR | 1.000 | 0.24% |
| Vitamin B1 (Thiamin) 100% VNR | 0.40 | 0.09% |
| Vitamin B2 (Riboflavine) 100% VNR | 0.51 | 0.12% |
| Vitamin B3 (Nicotinamide) 100% VNR | 5.87 | 1.38% |
| Vitamin B5 (Calcium D - Pantothenate) 100% VNR | 2.20 | 0.52% |
| Vitamin B6 (Pyridoxine) 100% VNR | 0.51 | 0.12% |
| Vitamin B8 (Biotin) 100% VNR 50µg | 0.02 | 0.005% |
| Vitamin B9 (A.Folique) 100% VNR | 0.07 | 0.02% |
| Vitamin B12 (Cyanocobalamine) 100% VNR | 0.0009 | 0.0002% |
| Zinc (Zn bisglycinate) 100%VNR | 16.67 | 3.92% |
| Gluconate Cuivre | 0.50 | 0.12% |
| Sodium Molybdate 1% 100% VNR | 2.500 | 0.59% |
| Selenium - L selenomethionine 100% VNR | 3.667 | 0.86% |
| *Banana flour as ecoulement agent* | 30.00 | 7.06% |
| *Total* | 425.07 mg | 100% |

Table 3 shows an exemplary hair care nutritional supplement composition in the form of a capsule comprising a total weight of 425.07 mg, wherein the capsule comprises 25.00mg Saw palmetto berries extract, 10.00mg of Fenugreek seed extract, 10.00mg of Ortieracine (nettleroot, Urticadioica) extract, 10.00mg of Ashwagandha root extract, 25.00mg Chrysanthellum indicum flower extract, 25.00 mg Rosemary (ursolicacid) Romarin extract, 20.00 mg Grape seed (Vitis viniferaOPC) extract, 20.00 mg Millet (methionine) Panicum miliaceum extract, 20.00mg Moringa seed extract, 30.00mg giant bamboo/Bambusa bamboo extract, 30.00mg Vitamin C 100% VNR 97.5%, 5.00mg Inositol, 3.30mg SOD Melon (super oxyde dismutase) extract, 50.00mg Co-enzyme 10, 50.00mg L- Arginine, 10.00mg L-Tyrosin, 10.00mg L LysineHCl, 2.75mg Vitamin K2 10000 ppm 100% VNR, 1mg Vitamin D3 (0,25%) 100% VNR, 0.40mg Vitamin B1 (Thiamin) 100% VNR, 0.51mg Vitamin B2 (Riboflavine) 100% VNR, 5.87mg Vitamin B3 (Nicotinamide) 100% VNR, 2.20mg Vitamin B5 (Calcium D - Pantothenate) 100% VNR, 0.51mg Vitamin B6 (Pyridoxine) 100% VNR, 0.02mg Vitamin B8 (Biotin) 100% VNR 50µg, 0.07mg Vitamin B9 (A.Folique) 100% VNR, 0.0009mg Vitamin B12 (Cyanocobalamine) 100% VNR, 16.67mg Zinc (Zn bisglycinate) 100%VNR, 0.50mg Gluconate Cuivre, 2.5mg Sodium Molybdate 100% VNR, 3.667mg Selenium - L selenomethionine 100% VNR, 30.00mg Banana flour as ecoulement agent.

Table 3 also shows an exemplary hair care nutritional supplement composition in the form of a capsule comprising 5.88% w/w saw palmetto berries extract; 2.35% w/w fenugreek seed extract. 2.35% w/w ortieracine ES(nettleroot, Urticadioica) extract; 2.35% w/w ashwagandha root extract; 5.88% w/w chrysanthellum indicum flower extract; 5.88% w/w rosemary leaf extract; 4.705% w/w grape seed extract; 4.705% w/w millet seed extract; 4.705% w/w moringa seed extract; 7.06% w/w bambusa bamboo extract; 8.26% w/w vitamin C 100% VNR; 1.18% w/w inositol; 0.78% w/w SOD melon (super oxyde dismutase); 11.76% w/w co-enzyme 10; 11.76% w/w L- arginine; 2.35% w/w L-tyrosin; 2.35% w/w L lysineHCl; 0.65% w/w vitamin K2 10000 ppm 100% VNR (75µg); 0.24% w/w vitamin D3 100% VNR; 0.09% w/w vitamin B1 (thiamin ) 100% VNR; 0.12% w/w vitamin B2 (riboflavine) 100% VNR; 1.38% w/w vitamin B3 (nicotinamide ) 100% VNR; 0.52% w/w vitamin B5 (calcium D - pantothenate) 100% w/w VNR; 0.12% w/w vitamin B6 (pyridoxine) 100% w/w VNR; 0.005% w/w vitamin B8 (biotin) 100% VNR 50µg; 0.02% w/w vitamin B9 (A.folique) 100% VNR; 0.0002% w/w vitamin B12 (cyanocobalamine) 100% VNR; 3.92% w/w zinc (Zn bisglycinate) 100%VNR; 0.12% w/w copper gluconate; 0.59% w/w sodium molybdate 100% VNR; 0.86% w/w selenium - L selenomethionine 100% VNR; and 7.06% w/w banana flour as ecoulement agent of the total weight of the capsule.

EXEMPLE 4: In another exemplary embodiment of the present invention a survey was conducted by taking feedback from 115 persons who used the oral composition of the present invention. The survey results of hair capsule tested 4 weeks, 8 weeks and 12 weeks over several persons out of which 115 participated in the survey to share their feedback.

The feedback received after daily consumption of the oral composition of the present invention in the form of a capsule over 4 weeks of regular use resulted that the supplement increased hair hydration by 24.83%, increased hair shine by 0.66%, reduced broken and damaged hair from brushing by 35.35%, increased hair count by 7.61%, decreased grey hair by 14.29%, increased hair shine from an average of 'dull, no reflectance' to '20% or less light reflectance.

The feedback received after daily consumption of the oral composition of the present invention in the form of a capsule over 8 weeks of regular use resulted that the supplement increased hair hydration by 53.21%, increased hair shine by 1.18%, reduced broken and damaged hair from brushing by 61.41%, increased hair count by 17.56%, decreased grey hair by 10%, increased hair shine from an average of'dull, no reflectance' to '60% or less light reflectance.

The feedback received after daily consumption of the oral composition of the present invention in the form of a capsule over 12 weeks of regular use resulted that the supplement increased hair hydration by 79.29%, increased hair shine by 1.80%, reduced broken and damaged hair from brushing by 86.06%, increased hair count by 34.07%, decreased grey hair by 2.86%, increased hair shine from an average of 'dull, no reflectance' to '80% or less light reflectance.

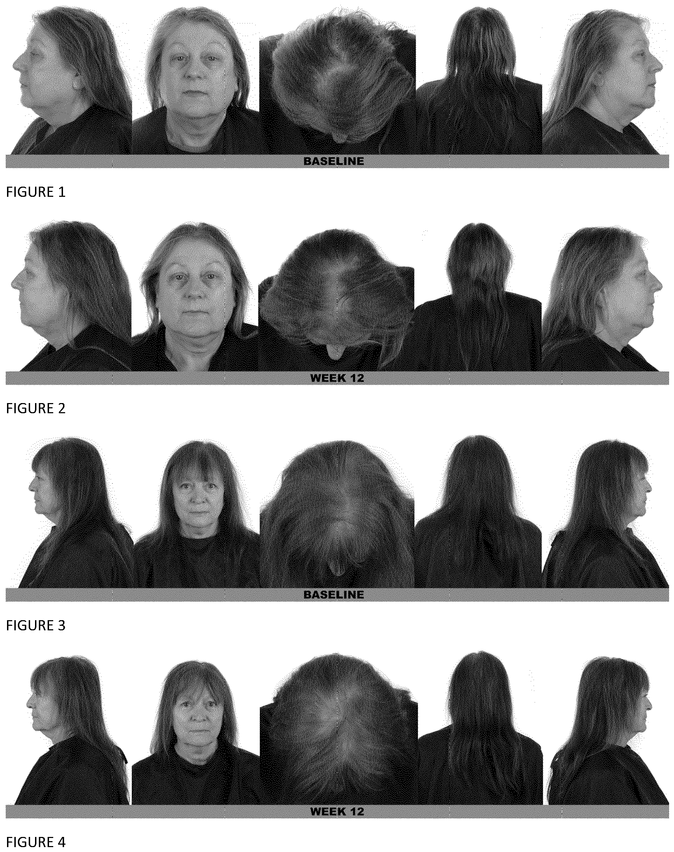

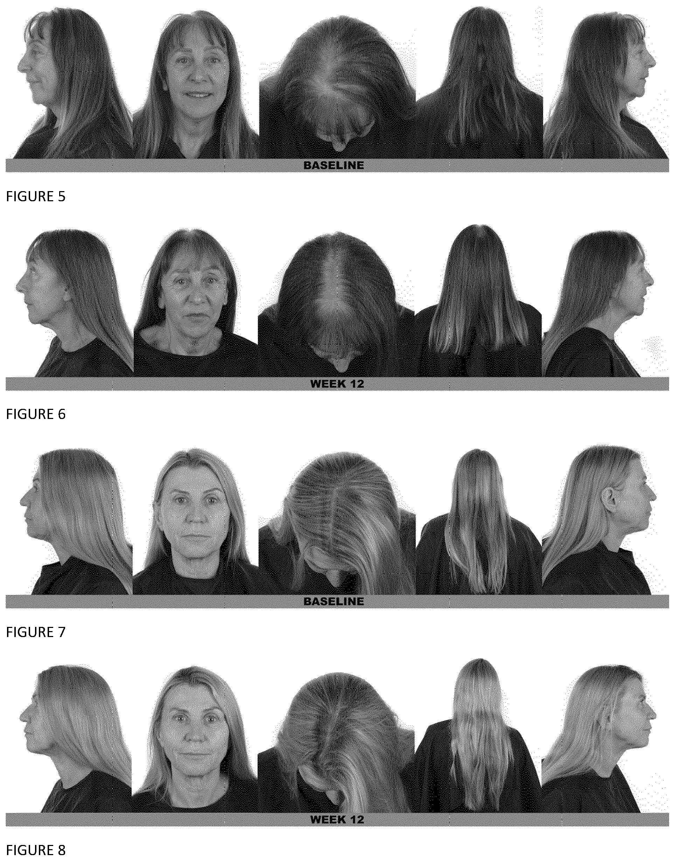

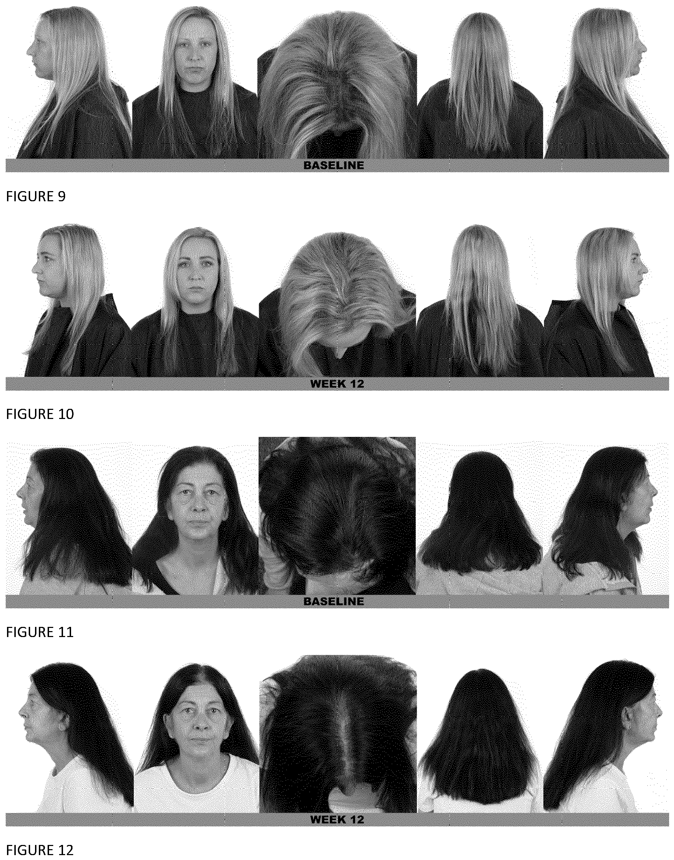

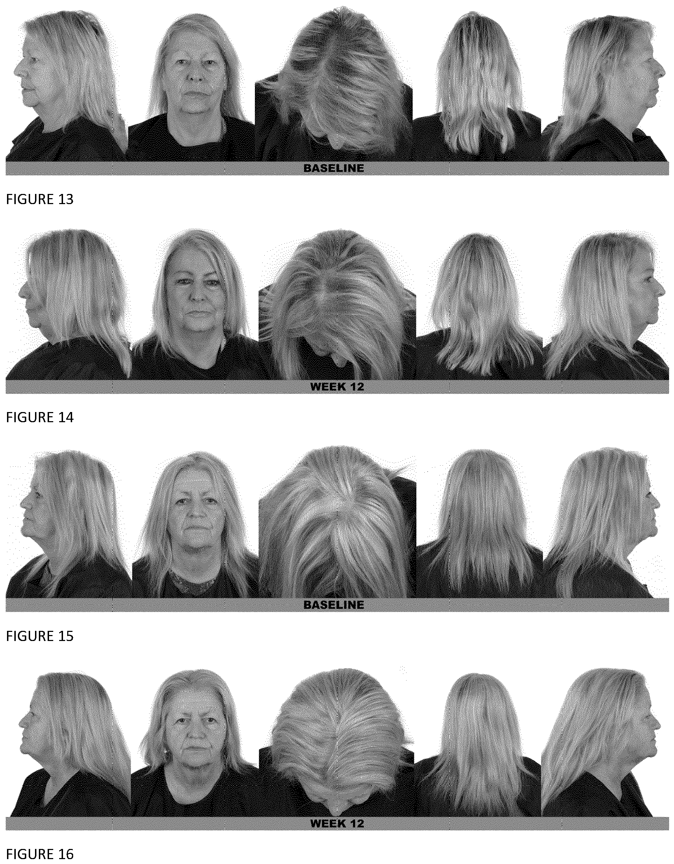

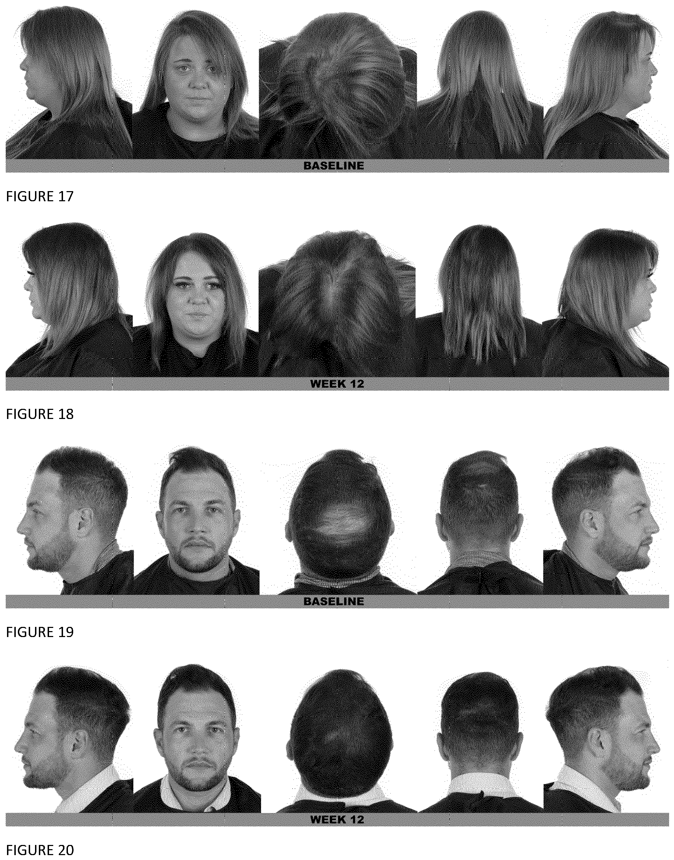

The results for 10 individuals are presented in figures 1 to 20. Each individual shows visible improvements in terms of reduction of hair loss, thinning hair problems, balding disorders and regeneration of hairs.

These and other advantages of the present invention will be apparent to those skilled in the art from the above specification. Accordingly, it will be recognized by those skilled in the art that changes or modifications may be made to the above-described embodiments without departing from the broad inventive concepts of the invention. Specific dimensions of any particular embodiment are described for illustration purposes only. It should therefore be understood that this invention is not limited to the particular embodiments described herein, but is intended to include all changes and modifications that are within the scope and spirit of the invention.

## Claims

1. A hair care oral nutritional supplement composition, comprising :
- fenugreek seed extract;
- ashwagandha root extract;
- bambusa bamboo extract;
- moringa seed extract.

2. The hair care oral nutritional supplement composition according to claim 1, further comprising:
- vitamin C;
- vitamin K2;
- zinc.

3. The hair care oral nutritional supplement composition according to claim 2, comprising:
- fenugreek seed extract in a range of 1.5 % to 7.8 % w/w;
- Ashwagandha root extract in a range of 1.5 % to 7.5 % w/w;
- bambusa bamboo extract in a range of 5.5 % to 11% w/w;
- moringa seed extract in a range of 3.5% to 10.5% w/w;
- vitamin C in a range of 7% to 13% w/w;
- vitamin K2 in a range of 0,28 % to 2 % w/w;
- zinc in a range of 3 % to 7 % % w/w.

4. The hair care oral nutritional supplement composition according to any one of the preceding claims, further comprising at least one, preferably all, of:
- saw palmetto berries extract;
- nettle root extract;
- chrysanthellum indicum flower extract;
- rosemary leaf extract;
- grape seed extract;
- millet seed extract;
- inositol;
- super oxyde dismutase melon.

5. The hair care oral nutritional supplement composition according to the preceding claim, comprising:
- saw palmetto berries extract in a range of 3.2% to 8.5% w/w;
- nettle root extract in a range of 1.3% to 5.6% w/w;
- chrysanthellum indicum flower extract in a range of 4.5% to 9.5% w/w;
- rosemary leaf extract in a range of 3.8% to 8.9% w/w;
- grape seed extract in a range of 3.1% to 7.2% % w/w;
- millet seed extract in a range of 2.5% to 8.2% w/w;
- inositol in a range of 0.7% to 3.5% w/w;
- super oxyde dismutase melon in a range of 0.3% to 2% w/w.

6. The hair care oral nutritional supplement composition according to any one of the preceding claims, wherein the composition further comprises at least one of : eucalyptus extract, pumpkin seed extract, green tea extract, licorice, reishi mushroom extract, eckolonia cava extract, tomato extract, or combination thereof.

7. The hair care oral nutritional supplement composition according to any one of the preceding claims, wherein the composition further comprises Lactoferrin.

8. The hair care oral nutritional supplement composition according to any one of the preceding claims, comprises botanicals extracts in a range of 15% to 50% w/w of the total weight, preferably wherein the botanical extracts are selected from at least one of: eckolonia cava extract, reishi mushroom extract, pumpkin seed extract, green tea extract, licorice, tomato extract, vitamin K2, vitamin C, lactoferrin, saw palmetto berries extract, fenugreek seed extract, nettle root extract, ashwagandha root extract, chrysanthellum indicum flower extract, rosemary leaf extract, grape seed extract, millet seed extracts, moringa seed extract, or combination thereof.

9. The hair care oral nutritional supplement composition according to any one of the preceding claims, wherein the composition further comprises L-Cysteine and/or choline.

10. The hair care oral nutritional supplement composition according to any one of the preceding claims, wherein the composition further comprises at least one of: co-enzyme 10, L- arginine, L-tyrosine, L-lysine HCl, vitamin D3, vitamin B1, vitamin B2, vitamin B3, vitamin B5, vitamin B6, vitamin B8, vitamin B9, vitamin B12, copper gluconate, sodium molybdate, selenium, banana flour or combination thereof.

11. The hair care oral nutritional supplement composition according to the preceding claim, comprising :
- 5.88% w/w saw palmetto berries extract;
- 2.35% w/w fenugreek seed extract;
- 2.35% w/w nettle root extract;
- 2.35% w/w ashwagandha root extract;
- 5.88% w/w chrysanthellum indicum flower extract;
- 5.88% w/w rosemary leaf extract;
- 4.705% w/w grape seed extract;
- 4.705% w/w millet seed extract;
- 4.705% w/w moringa seed extract;
- 7.06% w/w bambusa bamboo extract;
- 8.26% w/w vitamin C ;
- 1.18% w/w inositol;
- 0.78% w/w super oxyde dismutase melon;
- 11.76% w/w co-enzyme 10;
- 11.76% w/w L- arginine;
- 2.35% w/w L-tyrosine;
- 2.35% w/w L-lysine HCl;
- 0.65% w/w vitamin K2;
- 0.24% w/w vitamin D3 ;
- 0.09% w/w vitamin B1;
- 0.12% w/w vitamin B2;
- 1.38% w/w vitamin B3;
- 0.52% w/w vitamin B5;
- 0.12% w/w vitamin B6;
- 0.005% w/w vitamin B8;
- 0.02% w/w vitamin B9;
- 0.0002% w/w vitamin B12;
- 3.92% w/w zinc;
- 0.12% w/w copper gluconate;
- 0.59% w/w sodium molybdate;
- 0.86% w/w selenium;
- 7.06% w/w banana flour.

12. The hair care oral nutritional supplement composition according to any one of the preceding claims, wherein the oral composition is in a form selected from the group of: a tablet, a capsule, a candy, a pouch, a food supplement package, a toffee, a bar, a lozenz, sauce, juice or a combination thereof.

13. The hair care oral nutritional supplement according to the preceding claim, wherein the oral composition is in a form of a capsule, wherein the capsule comprises a capsule cover made of nano particles of gold or copper or a combination thereof with other bonding agents.

14. Use of a hair care oral nutritional supplement according to any one of the preceding claims for the non-therapeutic prevention and/or reduction of hair loss, thinning hair problems, balding disorders and regeneration of hairs in a natural way.

15. A hair care oral nutritional supplement according to any one of claims 1 to 13, for use in the prevention and/or reduction of hair loss, thinning hair problems, balding disorders and regeneration of hairs in a natural way.
